# EUROPÄISCHE PATENTSCHRIFT

(11) **EP 2 835 141 B1**
(45) Veröffentlichungstag und Bekanntmachung des Hinweises auf die Patenterteilung: **31.08.2016**
(21) Anmeldenummer: 14191464.8
(22) Anmeldetag: 13.04.2006
(51) Int. Cl.: A61M 1/10

(54) **Steuerung einer Blutpumpe**
Control of a blood pump
Commande de pompe à sang

(30) Priorität: 16.04.2005 DE 102005017546
(43) Veröffentlichungstag der Anmeldung: 11.02.2015
(62) Teilanmeldung aus: 06743298.9
(73) Patentinhaber: Abiomed Europe GmbH, 52074 Aachen (DE)
(72) Erfinder: Thorsten, Sieß, 52074 Aachen (DE); Rainer, Damen, 52074 Aachen (DE)
(74) Vertreter: Klunker . Schmitt-Nilson . Hirsch

(56) Entgegenhaltungen:
- WO-A-03/105669
- US-A- 4 846 152
- US-A- 6 139 487
- HIROFUMI ANAI ET AL: "AN APPROACH TO REDUCING HEMOLYSIS IN AN AXIAL-FLOW BLOOD PUMP", ASAIO JOURNAL, LIPPINCOTT WILLIAMS & WILKINS / ASAIO, HAGERSTOWN, MD, US, Bd. 41, Nr. 3, 1. Juli 1995 (1995-07-01), Seiten 771-774, XP000542964, ISSN: 1058-2916

## Beschreibung

Die Erfindung betrifft die Steuerung der Drehzahl einer rotatorischen Blutpumpe, die eine Auslegungsdrehzahl mit minimaler Ablösung und Verwirbelung der Blutströmung aufweist.

In EP 0 961 621 B1 (Impella) ist eine intravasale Blutpumpe beschrieben, die durch das Gefäßsystem in den Körper eingeführt und im Herzen oder an einer anderen Stelle, an der Blut gepumpt werden soll, platziert wird. Die Blutpumpe weist ein Gehäuse mit einem Durchmesser von 5,4 bis 6,4 mm auf, das den Stator eines Motors bildet. Der Rotor ist mit einem Impeller verbunden, der in einem Pumpenring rotiert. Diese sehr kleinformatige Blutpumpe hat eine relativ hohe Drehzahl in der Größenordnung von 30.000 U/min.

In EP 0 925 080 B1 (Impella) ist ein Verfahren zur Steuerung der Drehzahl einer Blutpumpe beschrieben, bei dem die Blutpumpe Drucksensoren aufweist, um die an der Blutpumpe herrschende Druckdifferenz zu ermitteln. In Abhängigkeit von den Signalen der Drucksensoren, wird die Motordrehzahl gesteuert. Dadurch wird ein gewünschter Volumenstrom erzeugt, um die gewünschte Pumpwirkung sicherzustellen.

In US 6,139,487 A ist eine Pumpvorrichtung mit zwei intrakardialen Blutpumpen beschrieben, die durch eine gemeinsame Steuereinheit gesteuert sind, so dass die Volumenströme beider Blutpumpen einander angeglichen werden.

Kontinuierlich fördernde rotatorische Blutpumpen haben eine sogenannte Auslegungsdrehzahl. Der das Blut vortreibende Impeller ist so geformt, dass bei der Auslegungsdrehzahl die Strömungsabrisse an den Impeller-Flügeln minimiert sind, oder idealerweise Strömungsabrisse überhaupt nicht auftreten. Dadurch wird eine turbulenzarme Strömung erreicht. Strömungsabrisse und Turbulenzen bewirken die Gefahr von Thrombenbildung, wobei Blut akkumuliert und an dem Impeller oder an anderen Stellen des Blutsystems anwächst. Dadurch wird zunächst die Funktion des Impellers verschlechtert. Außerdem besteht die Gefahr von Verstopfungen des Blutsystems.

Der Erfindung liegt die Aufgabe zugrunde, die Drehzahl einer rotatorischen Blutpumpe so zu steuern, dass permanent eine Blutförderung mit hoher Effektivität sichergestellt ist, wobei es möglich ist, die Blutpumpe zeitweise unterhalb der Auslegungsdrehzahl zu betreiben.

Die erfindungsgemäße Lösung weist die Merkmale des Patentanspruchs 1 auf. Hiernach erfolgt ein Betrieb der Blutpumpe mit wechselnden Drehzahlen, bei welchem abwechselnd eine niedrige Drehzahl und die Auslegungsdrehzahl eingenommen werden.

Die Erfindung basiert auf der Erkenntnis, dass eine Thrombenbildung am Impeller dadurch vermieden werden kann, dass mindestens zeitweise die Auslegungsdrehzahl eingenommen wird. Im Übrigen besteht aber die Möglichkeit, die Blutpumpe gefahrlos mit geringerer Drehzahl zu betreiben. Dadurch, dass jeweils nach einer gewissen Zeit des Betriebes mit geringer Drehzahl die Drehzahl auf die Auslegungsdrehzahl erhöht wird, werden Ansätze von Thrombenbildung am Impeller reformiert. Es hat sich gezeigt, dass ein nur kurzzeitiger Betrieb mit der Auslegungsdrehzahl ausreicht, um Ansätze von Thromben vom Impeller abzulösen, so dass ein Aufbau von Thromben vermieden wird. Die Erfindung erlaubt es, den Volumenstrom bzw. die Fördermenge unterhalb der Auslegungsdrehzahl zu wählen und erforderlichenfalls auch zu variieren, ohne dass ein ständiger Betrieb mit der hohen Auslegungsdrehzahl erforderlich wäre.

Die Erhöhung von der niedrigen Drehzahl auf die Aüslegungsdrehzahl erfolgt vorzugsweise mit einer Beschleunigung, die größer ist als 3.000 s⁻². Dadurch wird ein kurzzeitiger und rapider Anstieg der Pumperidrehzahl erreicht. Dies ist wegen der geringen Masse der Blutpumpe, die als intravasale oder parakardiale Blutpumpe ausgebildet ist, möglich.

Die Steuerung der Drehzahl der Blutpumpe erfolgt vorzugsweise durch Vorgabe einer Erregerfrequenz. Bei dem Rotor handelt es sich um einen schleifringlosen Gleichstrommotor, der im Stator zyklisch erregte Wicklungen aufweist und im Rotor mit Permanentmagneten versehen ist. Ein entfernt von dem Rotor angeordnetes Steuergerät liefert die Betriebsfrequenz für den Motor. Der Motor ist mit dem Steuergerät durch eine elektrische Leitung verbunden, die durch einen flexiblen Katheter hindurchführt.

Die Verlangsamung der Drehzahl bedarf einer Energiezufuhr. Sie erfolgt vorzugsweise durch ungebremstes natürliches Abklingen (Dämpfung durch den antreibenden Impeller), wobei die Verlangsamung ebenfalls größer ist als 3.000 s⁻² und somit innerhalb sehr kurzer Zeit erfolgt.

Im Rahmen der Erfindung ist es möglich, die rotatorische Blutpumpe nach einer ersten Verfahrensvariante zu betreiben, bei der periodisch Impulse mit der Auslegungsdrehzahl ausgeführt werden, und nach einer zweiten Variante, bei der die Beschleunigung und Verlangsamung fremdgesteuert durchgeführt werden.

Bei der ersten Variante wird die Förderung hauptsächlich durch die niedrige Drehzahl bestimmt und das Fördervolumen wird durch die kurzzeitigen Erhöhungen auf die Auslegungsdrehzahl nur unwesentlich beeinflusst. Dies hat zur Folge, dass die niedrige Drehzahl entsprechend dem Bedarf des Patienten eingestellt werden kann. Wenn sich das Herz des Patienten aufgrund der Unterstützung durch die Blutpumpe erholt hat und nunmehr weniger Unterstützung braucht, kann eine entsprechende niedrige Drehzahl der Blutpumpe gewählt werden, wobei die Entstehung von Thromben durch kurzzeitige Nadelimpulse vermieden wird, in denen die Drehzahl auf die Auslegungsdrehzahl erhöht wird. Diese Drehzahlerhöhungen beeinflussen die gesamte Fördermenge der Blutpumpe nur unwesentlich.

Bei der zweiten Variante kann mit der Blutpumpe, die eigentlich für eine kontinuierliche Förderung vorgesehen ist, eine pulsatile Herzunterstützung erfolgen. Der Pumpfunktion der Blutpumpe wird die natürliche Pumpfunktion des Herzens über-lagert. Hieraus ergibt sich ein periodisch veränderlicher Strom, wobei die periodische Veränderung durch die Pumpfunktion des Herzens hervorgerufen wird, durch die die Blutpumpe zeitweilig unterstützt wird, nämlich während der Systole, und zeitweilig gehemmt wird, nämlich während der Diastole. Die Erfindung ermöglicht es, durch Überwachung des Motorstroms die Zeiten hoher Pumpendrehzahl und niedriger Pumpendrehzahl mit der Pumpfunktion des Herzens zu synchronisieren bzw. die Herzunterstützung phasenversetzt zur Herzfunktion zu triggern, ohne dass eine EKG-Ableitung erforderlich wäre.

Die Erfindung ist insbesondere für die Rechtsherzunterstützung geeignet, bei der die Saugöffnung der Pumpe im rechten Atrium platziert wird, während die Pumpe in die Pulmonalarterie fördert.

Im Folgenden werden unter Bezugnahme auf die Zeichnungen Ausführungsbeispiele der Erfindung näher erläutert.

Es zeigen:
- Figur 1: eine schematische Darstellung des Aufbaus der Blutpumpe,
- Figur 2: in vergrößertem Maßstab die Strömungsverhältnisse an einem Flügel des Impellers der Pumpe,
- Figur 3: den zeitlichen Verlauf der Pumpendrehzahl bei periodischer Steuerung,
- Figur 4: den zeitlichen Verlauf der Pumpendrehzahl bei einem Pumpenbetrieb, der mit der Herzfunktion synchronisiert ist, und
- Figur 5: eine schematische Darstellung des Pumpenantriebs und des Sensors zur Ermittlung des Motorstroms.

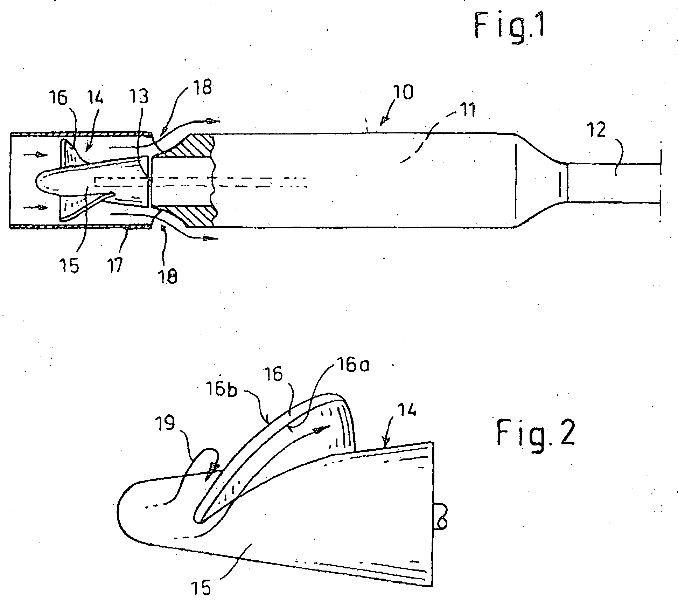

In der Figur 1 ist eine rotatorische Blutpumpe 10 zur Herzunterstützung dargestellt. Die Blutpumpe ist hier eine intravasale Blutpumpe, deren maximaler Außendurchmesser an keiner Stelle größer ist als 7 mm. Die Blutpumpe kann aber auch als intrakardiale Blutpumpe ausgebildet sein, die durch eine Inzisionsstelle des Herzens hindurch geführt wird. Eine derartige Blutpumpe kann einen etwas größeren Durchmesser haben. Ferner kann die Pumpe parakardial (um das Herz herum) platziert werden und über zwei Inzisionsstellen Blut vor oder aus dem Herzen entnehmen und hinter dem Herzen durch die zweite Inzisionsstelle wieder zuführen.

Die Blutpumpe 10 weist einen langgestreckten zylinderischen Motor 11 auf, an dessen proximales Ende sich ein flexibler Katheter 12 anschließt. Durch den Katheter 12 gehen (nicht dargestellte) elektrische Leitungen hindurch, die zu einem Steuergerät führen.

Die Welle 13 des Motors 11 trägt einen Impeller 14 mit einer zum distalen Ende hin spitz zulaufenden Nabe 15, von der schraubenförmige Flügel 16 abstehen. Der Impeller 14 ist von einem länglichen Ring 17 umgeben, der etwa den gleichen Außendurchmesser hat wie der Motor 11. Zwischen dem Motor 11 und dem Ring 17 befinden sich Ausströmöffnungen 18. Durch Rotation des Impellers 14 saugt dieser axial Blut an und fördert es in Richtung auf den Motor 11. Das Blut verlässt die Blutpumpe durch die Öffnungen 18, um anschließend an dem Motor 11 entlang zu strömen. Die in Figur 1 dargestellte Pumpe kann ebenfalls so strömungstechnisch ausgelegt werden, dass eine umgekehrte Flussrichtung erreicht wird.

Figur 2 zeigt eine schematische vergrößerte Darstellung eines Teils des Impellers 14. Von der Nabe 15 steht ein Flügel 16 ab, der nach Art eines Flugzeugflügels gebogen ist. Infolge der Strömung entsteht an der konkaven Innenseite 16a ein positiver Druck und entlang der konvexen Oberseite 16b entsteht ein Unterdruck oder negativer Druck. Die Flügel 16 sind so gestaltet, dass bei einer Auslegungsdrehzahl eine abrissfreie Strömung entlang der Flügel entsteht. Bei dem vorliegenden Ausführungsbeispiel beträgt die Auslegungsdrehzahl 30.000 U/min. Bei niedrigeren Drehzahlen entstehen Strömungsabrisse und Wirbel 19, die eine Blutschädigung durch Entstehung von Thromben bewirken können. Die Thromben wachsen auf der Oberfläche des Flügels 16 und beeinträchtigen die Pumpenströmung, wodurch die Thrombenbildung noch weiter verstärkt wird.

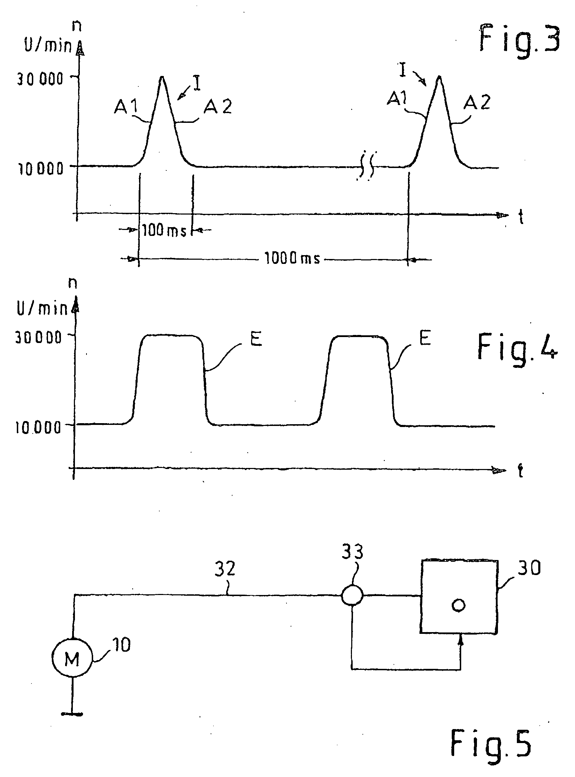

Figur 3 zeigt ein Diagramm eines zeitlichen Verlaufs der Drehzahl n des Motors 11, wobei längs der Abszisse die Zeit t und längs der Ordinate die Drehzahl n in Umdrehungen pro Minute aufgetragen sind. Die Drehzahl kann durch entsprechende Einstellung des Steuergerätes variiert werden. Bei dem vorliegenden Beispiel ist angenommen, dass die Drehzahl generell 10.000 U/min betragen soll. Dieser Wert entspricht beispielhaft der Fördermenge, die der Patient zur Herzunterstützung in der Entwöhnungsphase benötigt.

Die kontinuierliche Drehzahl von 10.000 U/min wird unterbrochen durch periodisch auftretende Impulse I, in denen die Drehzahl kurzzeitig auf die Auslegungsdrehzahl von 30.000 U/min ansteigt. Die Anstiegsflanke A1 des Impulses I hat eine Steigung von mehr als 3.000 s⁻². In ihr steigt die Drehzahl des Motors kontinuierlich von dem niedrigen Wert von 10.000 U/min auf die Auslegungsdrehzahl von 30.000 U/min an. Dieser Frequenzanstieg wird durch das Steuergerät erzeugt. Nach Erreichen der Auslegungsdrehzahl klingt die Drehzahl in einer Abstiegsflanke A2 ab. Dieser Abfall erfolgt ohne Zuführung von Bremsenergie oder anderweitiger externer Energie durch Unterlassung der Zufuhr von Antriebsenergie. Auch das Abklingen erfolgt mit einer Verzögerung von mehr als 3.000 s⁻². Das Abklingen wird beendet, wenn die Drehzahl wieder den unteren Wert erreicht hat, der anschließend beibehalten wird. Die Dauer der Impulse I beträgt bei dem vorliegenden Ausführungsbeispiel etwa 100 ms und die Periodendauer, also die Dauer eines Impulses und einer anschließenden Impulslücke, beträgt etwa 1.000 ms.

Die Impulse I sind Nadelimpulse mit im Verhältnis zur Periodendauer kurzer Impulsbreite. Daher ist auch die zusätzlich Förderleistung, die durch die Impulse I erbracht wird, im Verhältnis zu der Grund-Förderleistung bei 10.000 U/min klein. Diese Grund-Förderleistung wird durch die Impulse I also wenig beeinflusst. Die Impulse I haben die Wirkung, dass trotz eines geringen Flusses Thrombenbildungen am Impeller selbst über Tage hinweg vermieden werden.

Figur 4 zeigt eine zweite Variante des erfindungsgemäßen Verfahrens. Bei dieser wird ebenfalls die Drehzahl n durch Impulse E vorübergehend erhöht, jedoch sind die Impulse E wesentlich breiter als die Impulse I nach der ersten Verfahrensvariante. Die Impulse E, in denen die Drehzahl vorübergehend erhöht wird, werden synchron mit der Herzfrequenz erzeugt, jedoch hierzu phasenversetzt. Figur 5 zeigt die Schaltung des Motors 11 und eines Steuergerätes 30, das die Frequenz und den Strom für den Motor 11 liefert. Das Steuergerät 30 ist mit dem Motor über eine Leitung 32 verbunden. Ein Sensor 33 misst den in der Leitung 32 fließenden Strom und teilt diesen dem Steuergerät 30 mit. Der Motorstrom ist abhängig von der Last des Motors. Die Last ändert sich über der Zeit entsprechend der natürlichen Pumpwirkung des Herzens, die der kontinuierlichen Pumpwirkung der Pumpe 10 überlagert ist. Aus dem zeitlichen Verlauf des Stromes, der von Sensor 33 gemessen wird, kann die Zeitsteuerung für die Erzeugung der Impulse E in Figur 4 abgeleitet werden. Dadurch wird eine EKG-Ableitung mit zusätzlichen Messelektroden vermieden. Die Pumpe benötigt keine externen Sensoren.

Gemäß Figur 4 erfolgt der Betrieb der Pumpe auch bei dieser Variante des Verfahrens in der Weise, dass entweder eine niedrige Drehzahl n oder die Auslegungsdrehzahl eingestellt ist. Die Beschleunigung der Pumpe und die Verlangsamung betragen auch hier mehr als 3.000 s⁻². Bei dieser Variante des Pumpenbetriebes wird mit der rotatorischen Blutpumpe ein pulsierender Pumpenbetrieb erzeugt, der mit der natürlichen Pumpfrequenz des Herzens abgestimmt ist.

## Patentansprüche

1. System, umfassend eine rotatorische Blutpumpe (10) die eine Auslegungsdrehzahl mit minimaler Ablösung und Turbulenz der Blutströmung aufweist; und eine Steuerung,
**dadurch gekennzeichnet, dass** die Steuerung eingerichtet ist, die Drehzahl der Blutpumpe so zu steuern, dass ein Betrieb der Blutpumpe mit wechselnden Drehzahlen erfolgt, wobei abwechselnd eine niedrige Drehzahl und die Auslegungsdrehzahl eingenommen werden.

2. System nach Anspruch 1, wobei eine Erhöhung von der niedrigen Drehzahl auf die Auslegungsdrehzahl mit einer Beschleunigung erfolgt, die größer ist als 3.000 s⁻².

3. System nach Anspruch 1 oder 2, wobei eine Verlangsamung von der Auslegungsdrehzahl auf die niedrige Drehzahl durch ungebremstes natürliches Abklingen erfolgt.

4. System nach Anspruch 3, wobei die Verlangsamung größer ist als 3.000 s⁻².

5. System nach einem der Ansprüche 1 bis 4, wobei das System eingerichtet ist, den Motorstrom der Blutpumpe (10) zu messen, daraus der Rhythmus einer überlagerten Pumpfunktion eines Herzens zu bestimmen und den Betrieb der Blutpumpe mit der überlagerten Pumpfunktion zu synchronisieren.

6. System nach Anspruch 5, wobei die Steuerung eingerichtet ist, die Pumpendrehzahl mit der Pumpfunktion in Phase oder phasenversetzt zu synchronisieren.

7. System nach einem der Ansprüche 1 bis 6, wobei die Steuerung eingerichtet ist, einen pulsierenden Betrieb der Blutpumpe zu erzeugen, der mit der natürlichen Pumpfrequenz eines Herzens abgestimmt ist.

8. System nach Anspruch 6 oder 7, wobei die Steuerung eingerichtet ist, einen Betrieb der Blutpumpe zu erzeugen, der ohne EKG-Ableitung erfolgt.

9. System nach einem der Ansprüche 1 bis 8, umfassend einen elektrischen Motor (11), der über einen Katheter (12) mit einem Steuergerät (30) verbunden ist, und einen von dem Motor (11) angetriebenen Impeller (14).

10. System nach einem der Ansprüche 1 bis 9, umfassend einen Sensor (33), welcher eingerichtet ist, einen Motorstrom zu messen, wobei die Steuerung eingerichtet ist, die Pumpendrehzahl in Abhängigkeit von den Signalen des Sensors (33) zu variieren.

11. System nach einem der Ansprüche 1 bis 4 oder 9, wobei das System eingerichtet ist, periodische Impulse (I) mit der Auslegungsdrehzahl durchzuführen, wobei die Periodendauer mindestens fünf Mal so lang ist wie die Breite der Impulse (I).

12. System nach einem der Ansprüche 1 bis 11, wobei das System so eingerichtet ist, dass die niedrige Drehzahl entsprechend dem Bedarf eines Patienten eingestellt werden kann.

## Claims

1. A system including a rotary blood pump (10) having a design rotational speed with minimal detachment and swirling of the blood flow; and a control,
**characterized in that** the control is adapted to control the rotational speed of the blood pump such that an operation of the blood pump is effected at alternating rotational speeds, wherein a low rotational speed and the design rotational speed are alternately reached.

2. The system according to claim 1, wherein an increase from the low rotational speed to the design rotational speed takes place at an acceleration of more than 3,000 s².

3. The system according to claim 1 or 2, wherein a deceleration from the design rotational speed to the low rotational speed takes place by a non-braked natural slowdown.

4. The method according to claim 3, wherein the deceleration exceeds 3,000 s².

5. The system according to any of the claims 1 to 4, wherein the system is adapted to measure the motor current of the blood pump (10), to ascertain therefrom the rhythm of a superimposed pumping function of a heart, and to synchronize the operation of the blood pump with the superimposed pumping function.

6. The system according to claim 5, wherein the control is adapted to synchronize the pump's rotational speed with the pumping function in phase or with a phase shift.

7. The system according to any of the claims 1 to 6, wherein the control is adapted to generate a pulsating operation of the blood pump which is coordinated with the natural pumping frequency of a heart.

8. The system according to claim 6 or 7, wherein the control is adapted to generate an operation of the blood pump which takes place without ECG recording.

9. The system according to any of the claims 1 to 8, including an electric motor (11) connected to a control device (30) via a catheter (12) and an impeller (14) driven by the motor (11).

10. The system according to any of the claims 1 to 9, including a sensor (33) adapted to measure a motor current, wherein the control is adapted to vary the pump's rotational speed in dependence on the signals of the sensor (33).

11. The system according to any of the claims 1 to 4 or 9, wherein the system is adapted to perform periodic pulses (I) with the design rotational speed, wherein the period duration is at least five times as long as the width of the pulses (I).

12. The system according to any of the claims 1 to 11, wherein the system is adapted such that the low rotational speed can be adjusted according to the needs of a patient.

## Revendications

1. Système comprenant une pompe à sang (10) rotative ayant une vitesse de rotation nominale à séparation de flux et turbulence de l'écoulement sanguin minimales; et une commande,
**caractérisé en ce que** la commande est conçue pour commander la vitesse de rotation de la pompe à sang de telle façon qu'un fonctionnement de la pompe à sang a lieu à des vitesses de rotation qui s'alternent, une faible vitesse de rotation et la vitesse de rotation nominale étant adoptées en alternance.

2. Système selon la revendication 1, l'augmentation de la faible vitesse de rotation jusqu'à obtention de la vitesse de rotation nominale ayant lieu à une accélération supérieure à 3.000 s^{.2}.

3. Système selon la revendication 1 ou 2, un ralentissement de la vitesse de rotation nominale jusqu'à obtention de la faible vitesse de rotation ayant lieu par régressement naturel non freiné.

4. Système selon la revendication 3, le ralentissement étant supérieur à 3.000 s^{.2}.

5. Système selon une des revendications de 1 à 4, le système étant conçu pour mesurer le courant de moteur de la pompe à sang (10), pour déterminer à partir de cela le rythme d'une fonction de pompe superposée d'un coeur et pour synchroniser le fonctionnement de la pompe à sang avec la fonction de pompe superposée.

6. Système selon la revendication 5, la commande étant conçue pour synchroniser en phase ou de manière déphasée la vitesse de rotation de la pompe avec la fonction de pompe.

7. Système selon une des revendications de 1 à 6, la commande étant conçue pour générer un fonctionnement pulsé de la pompe à sang accordé avec la fréquence naturelle de pompe d'un coeur.

8. Système selon la revendication 6 ou 7, la commande étant conçue pour générer un fonctionnement de la pompe à sang ayant lieu sans tracé ECG.

9. Système selon une des revendications de 1 à 8, comprenant un moteur électrique (11) relié, par l'intermédiaire d'un cathéter (12), à un appareil de commande (30), et une turbine (14) entraînée par le moteur (11).

10. Système selon une des revendications de 1 à 9, comprenant un capteur (33) conçu pour mesurer un courant de moteur, la commande étant conçue pour varier la vitesse de rotation de la pompe en fonction des signaux du capteur (33).

11. Système selon une des revendications de 1 à 4 ou 9, le système étant conçu pour effectuer des impulsions périodiques (I) à la vitesse de rotation nominale, la duré de période étant au moins cinq fois plus longue que la largeur des impulsions (I).

12. Système selon une des revendications de 1 à 11, le système étant conçu de telle façon que la faible vitesse de rotation peut être réglée en fonction du besoin d'un patient.
